# EUROPEAN PATENT APPLICATION

(11) **EP 1 698 327 A1**
(43) Date of publication of application: **06.09.2006**
(21) Application number: 06386001.9
(22) Date of filing: 02.02.2006
(51) Int. Cl.: A61K 9/00, A61K 31/495

(54) **Stable pharmaceutical compositions of itraconazole in aqueous environment**

(30) Priority: 09.02.2005 GR 20050100058
(71) Applicant: Verisfield (UK) Ltd., 115 28 Athens (GR)
(72) Inventor: Avgoustakis, Constantinos, 26442 Patras (GR)

(57) **Abstract**

In the present invention a novel pharmaceutical composition, which contains Itraconazole as an active ingredient and in which a range of celluloses are used as stabilizing agents for the preparation of oral aqueous solutions of Itraconazole, is described.

## Description

### Background of the invention

The solubility problems of the azoles like Itraconazole, a substance described in the patent US 4, 267, 179, could be overcome by the preparation of suitable pharmaceutical forms and the use of excipients, which facilitate the dissolution of these substances and enhance the stability of the resultant solutions, without affecting their structure and bioavailability. For the preparation of aqueous oral pharmaceutical preparations of Itraconazole, complexing agents like cyclodextrines have been used which transform Itraconazole into water-soluble complexes. Patent PCT/EP94/03169 discloses the composition of the commercially available aqueous oral form of Itraconazole (SPORANOX, 10 mg/ml), using the derivative of cyclodextrin - hydroxypropyl-β-cyclodextrin, for the solubilisation of Itraconazole through the formation of complexes. However the use of cyclodextrin derivatives increases the cost of the formulation and raises safety issues since cyclodextrins can cause toxicity in humans.

The composition of the present invention excludes the use of cyclodextrins and proposes the use of a cellulose derivative as a stabilizing agent for Itraconazole in the aqueous solution. Patent EP 1103262 discloses the composition of an oral solution of an antifungal agent like Itraconazole, making use of strong acids like hydrochloric acid or sulfuric acid or acetic acid for the solubilisation of Itraconazole. The composition also includes an alcoholic co-solvent like propylene glycol or ethanol or glycerol for the dilution of the prepared solution. These oral liquid preparations these aqueous forms suffer from reduced stability. The proposed composition in the present patent application makes use of a cellulose derivative as a stabilizing agent for Itraconazole and also an acid - hydrochloric acid or nitric acid or phosphoric acid or sulphuric acid, as a pH regulator, and an alcoholic co-solvent (propylene glycol) as a common excipient for the orally administered liquid preparations.

Moreover, the patent WO 96/19186 of Johnson & Johnson Consumer Products Inc, discloses the composition of Itraconazole, for topical administration to the nail, using special compounds (urea, amino acids, e.t.c.), which enhance the permeation of the antifungal agent through the nail tissue. The said patent also discloses the use of propylene glycol as a solvent in the proposed formulations and the use of hydroxypropyl-cellulose (HPC) as a thickening agent. The use of hydroxypropyl-cellulose in the proposed composition of the present invention has a different role, as it is used as a stabilizing agent for Itraconazole in the aqueous solution. Moreover, the use of propylene glycol in the present invention has the role of a common excipient used in orally administered liquid preparations and does not contribute to the proposed formulation.

### Description of the invention

The present invention is concerned with novel composition of an aqueous oral form of the antifungal active ingredient Itraconazole.

Itraconazole is a potent antifungal agent with activity against surface and systemic fungal infections. Itraconazole has a very low solubility in water (s<1 µg /ml), which makes difficult the preparation of aqueous solutions appropriate for oral administration of Itraconazole.

In the present invention, a variety of celluloses (Table 2) in proportions 0.01% - 0.8% w/v, are applied for the stabilization of aqueous oral forms of Itraconazole, which are based on conventional excipients for aqueous oral forms, such as Propylene glycol, which acts as a co-solvent of the active ingredient.

The presence of these celluloses is necessary for the formation of a stable aqueous solution of Itraconazole.

The mechanism of stabilization is considered to be based on the formation of hydrogen bonds between the molecules of Itraconazole and the polymeric chains of the celluloses (Table 2). The proposed oral liquid forms of Itraconazole according to the present invention may also contain sweetening and flavouring agents.

The pH of the aqueous forms of Itraconazole according to the present invention ranges between 1.2 - 2.0.

In the oral liquid formulations of Itraconazole according to the present invention, the following ingredients are used:

### Oral solution

a. Itraconazole, as the active ingredient.
b. Celluloses (Table 2), as stabilizing agents of the aqueous solution.
c. Propylene glycol, as an alcoholic co-solvent.
d. Sorbitol, as a sweetening agent.
e. Sodium saccharin, as a sweetening agent.
f. Acids (Table 1) as acidifying and pH adjusting agents.
g. Flavouring agents, as taste improving agents.
h. 2N sodium hydroxide solution [NaOH (2N)] or other inorganic or organic bases, as pH adjusting agents

### Method of preparation of oral solution

a. All the required quantities of any of the celluloses stated in Table 2 and Sodium saccharin are weighed and transferred to the main tank of preparation, which is equipped with a stirrer.
b. Quantity of water equal to half of the total required is added and the mixture is left under stirring in a covered container for approximately 20 hours, until the cellulose is completely dissolved.
c. The total required quantity of Propylene glycol is transferred to the main container where the oral solution will be prepared.
d. The required quantity of any of the acids stated in Table 1 is added to Propylene glycol and is left for 1-2 minutes under stirring.
e. The whole quantity of Itraconazole is added to the mixture of Propylene glycol and the acid slowly and under stirring. Stirring is continued until Itraconazole is completely dissolved (2 hours approximately).
f. When Itraconazole is completely dissolved, the solution of the cellulose and Sodium saccharin (stage a) is added slowly and under intense stirring.
g. The mixture is left under stirring for 5-10 min.
h. Addition of the required quantity of Sorbitol 70% slowly and under intense stirring.
i. The mixture is left under stirring for 5-10 min.
j. Addition of flavouring agents, under stirring
k. Addition of the remaining quantity of water very slowly and under intense stirring.
1. The resulting solution has pH (1.5-1.8)
m. If required, the pH can be adjusted with 2N NaOH solution.

**Table 1: Acids that can be used in the preparation of Itraconazole oral aqueous solution.**

| |
|---|
| Hydrochloric acid (HCL 37%) |
| Nitric acid |
| Phosphoric acid |
| Sulfuric acid |

**Table 2: Celluloses that can be used in the preparation of Itraconazole oral aqueous solution.**

| |
|---|
| Hydroxypropylmethylcellulose (HPMC) |
| Hydroxypropylcellulose (HPC) |
| Carboxymethylcellulose calcium |
| Carboxymethylcellulose sodium |
| Hydroxyethylcellulose |
| Methylcellulose |
| Microcrystalline cellulose |
| Cellulose ethers |
| Cellulose esters |
| Oxidised cellulose |

### Example 1

Required quantities for the production of 1000 ml of oral solution.

| **Ingredients** | **Use** | **Quantity** |
|---|---|---|
| Itraconazole | Active ingredient | 10.00g |
| Propylene glycol | Alcoholic co-solvent | 225.00g |
| Anyone of the acids stated in Table 1 | pH adjusting agent | qs to dissolve itraconazole) |
| Anyone of the celluloses stated in table 2 | Stabilising agent | 2.25g |
| Sorbitol (70%) | Sweetening agent | 300.00g |
| Sodium Saccharin | Sweetening agent | 0.60g |
| Cherry flavour | Taste improving agent | 0.75 g |
| Caramel flavour | Taste improving agent | 0.20g |
| 2N Sodium hydroxide solution | pH adjusting agent | q.s. to pH 1.2 - 2.0 |
| Water | Carrier and diluting agent | q.s. to 1000 ml |

### Example 2

Required quantities for the production of 1000 ml of oral solution

| **Ingredients** | **Use** | **Quantity** |
|---|---|---|
| Itraconazole | Active ingredient | 10.00g |
| Propylene glycol | Alcoholic co-solvent | 550.00g |
| Anyone of the acids stated in Table 1 | pH adjusting agent | qs to dissolve itraconazole |
| Anyone of the celluloses stated in Table 2 | Stabilising agent | 1.25g |
| Sorbitol (70%) | Sweetening agent | 300.00g |
| Sodium Saccharin | Sweetening agent | 0.60g |
| Cherry flavour | Taste improving agent | 0.75mg |
| Caramel flavour | Taste improving agent | 0.30mg |
| 2N Sodium hydroxide Solution | pH adjuster | q.s. to pH 1.2 - 2.0 |
| Water | Carrier and diluting agent | q.s. to 1000 ml |

## Claims

1. A pharmaceutical composition in aqueous form only for oral administration which contains Itraconazole as the active ingredient, and a cellulose - Hydroxy-propylmethylcellulose (HPMC) or Hydroxy-propylcellulose (HPC) or Carboxymethyl-cellulose calcium or Carboxymethyl-cellulose sodium or Hydroxy-ethylcellulose or Methyl-cellulose or Microcrystalline cellulose or Cellulose ethers or Cellulose esters or Oxidised cellulose as a stabilizing agent, and moreover an alcoholic co-solvent - Propylene glycol, an acid - Hydrochloric acid 37% or Nitric acid or Phosphoric acid or Sulfuric acid and other common excipients for aqueous oral pharmaceutical forms like: flavouring and sweetening agents as taste improving agents.

2. A pharmaceutical composition according to claim 1, for the aqueous and only orally administered forms, which contains 1 mg/ml to 10mg/ml Itraconazole as active ingredient, 0.1 mg/ml to 8.0 mg/ml cellulose - Hydroxypropylmethyl-cellulose (HPMC) or Hydroxypropylcellulose (HPC) or Carboxymethylcellulose calcium or Carboxymethylcellulose sodium or Hydroxy-ethylcellulose or Methylcellulose or Microcrystalline cellulose or Cellulose ethers or Cellulose esters or Oxidised cellulose as a stabilizing agent, 100 mg/ml to 1000 mg/ml Propylene glycol as alcoholic co-solvent and diluting agent.

3. A pharmaceutical composition according to claim 2, for aqueous and only orally administered forms, which has a pH ranging between 1.2 and 2.0.

4. A pharmaceutical composition according to claim 2, for the aqueous and only orally administered forms, which has a pH ranging between 1.3 and 2.0.

5. A pharmaceutical composition according to claim 2, for the aqueous and only orally administered forms, which has a pH ranging between 1.4 and 1.8.

6. A pharmaceutical composition according to claims 2 and 5 for the aqueous and only orally administered forms, which contains one or more sweetening agents like Sodium saccharin or/and Sorbitol (70%).
